# EUROPÄISCHE PATENTSCHRIFT

(11) **EP 3 014 005 B1**
(45) Veröffentlichungstag und Bekanntmachung des Hinweises auf die Patenterteilung: **09.08.2017**
(21) Anmeldenummer: 14730153.5
(22) Anmeldetag: 12.06.2014
(51) Int. Cl.: C08L 5/08, D04H 1/4274, A61L 27/00, D04H 1/728, C08L 89/06, A61L 27/56, A61L 27/24

(54) **DREIDIMENSIONALE, PORÖSE STRUKTUR AUS NANOFASERVLIES-FRAGMENTEN UND VERFAHREN ZU DEREN HERSTELLUNG**
THREE-DIMENSIONAL POROUS STRUCTURE MADE OF NANOFIBRE WEB FRAGMENTS AND METHOD FOR THE PRODUCTION THEREOF
STRUCTURE POREUSE TRIDIMENSIONNELLE COMPOSÉE DE FRAGMENTS DE NANO-NON-TISSÉ ET SON PROCÉDÉ DE PRODUCTION

(30) Priorität: 28.06.2013 DE 102013212699
(43) Veröffentlichungstag der Anmeldung: 04.05.2016
(73) Patentinhaber: Fraunhofer-Gesellschaft zur Förderung der angewandten Forschung e.V., 80686 München (DE); SpinPlant GmbH, 04103 Leipzig (DE)
(72) Erfinder: HENNING, Sven, 06114 Halle (Saale) (DE); HEILMANN, Andreas, 09573 Erdmannsdorf/Augustusburg (DE); SCHWAN, Stefan, 06114 Halle (Saale) (DE); FRIEDMANN, Andrea, 04539 Groitzsch (DE); MEISEL, Hans Jörg, 14163 Berlin (DE); GANEY, Timothy, Tampa, Florida 33604 (US); HERBST, Christian, 14163 Berlin (DE); HILLRICHS, Georg, 37083 Göttingen (DE)
(74) Vertreter: Pfenning, Meinig & Partner mbB
(86) Internationale Anmeldenummer: PCT/EP2014/062258
(87) Internationale Veröffentlichungsnummer: WO 2014/206755

(56) Entgegenhaltungen:
- EP-A1- 0 371 847
- EP-A1- 0 603 774
- WO-A2-2007/132186
- WO-A2-2012/006072
- US-A1- 2002 090 725

## Beschreibung

Es wird eine dreidimensionale, poröse Struktur aus Fragmenten eines Nanofaservlieses bereitgestellt. Darüberhinaus wird ein Verfahren zur Herstellung einer dreidimensionalen, porösen Struktur aus Nanofaservlies-Fragmenten vorgeschlagen. Die dreidimensionale, poröse Struktur findet beispielsweise Anwendung in der Medizin, bevorzugt der regenerativen Medizin. Darüberhinaus kann die erfindungsgemäße Struktur aus Fragmenten eines Nanofaservlieses zur Behandlung von Gewebeschäden, zur Immobilisierung von biologischen Zellen, zur Konstruktion von biologischem Gewebe und als biologischer Füllstoff *in vitro* als auch *in vivo* verwendet werden.

Die Anwendung isolierter, elektrogesponnener Nanofasern für den Aufbau von künstlichem biologischem Gewebe ist bereits aus der US 2002/009725 A1, der WO 2007/132186 A2 und der WO 2012/006072 A2 bekannt.

Aber auch Vliese, Matten oder Filze (Non-Wovens) aus Nanofasern zeigen außergewöhnliche Eigenschaften wie eine große spezifische Oberfläche, eine einstellbare Porosität und die Möglichkeit zur Ausrüstung mit therapeutisch wirksamen

Substanzen, die solche Nanovliese für Anwendungen in der Medizin interessant machen (Kittelmann, W, "Vliesstoffe: Rohstoffe, Herstellung, Anwendung, Eigenschaften, Prüfung", Wiley-VCH,Weinheim, 2000; Zahedia, P. et al, Polym. Adv. Technol., 2010, Band 21, S. 77-95; Schofer, M.D. et al., PLoS One, 2011, Band 6, S. 9)

In den letzten Jahren trat dabei besonders die Entwicklung und Erzeugung von Nanovliesen mit Faserdurchmessern von einigen 10 Nanometern bis einigen Mikrometern durch das Elektrospinnen in den Mittelpunkt. Inzwischen werden diese Vliese aus elektrogesponnenen Nanofasern kommerziell gefertigt und bereits in geringem Umfang für verschiedene Einsatzfalle, z.B. als Bestandteil von Implantaten, genutzt.

Die Optimierung der Fasereigenschaften ist bezüglich Materialauswahl, Faserdurchmesser und Faserlange weit fortgeschritten, auch sind inzwischen stabile Wirkstoffeinlagerungen in die Fasern und auch die Einarbeitung von Vernetzungsmitteln gelungen. Derzeit verfügbare Vliesmaterialien werden typischerweise als dünne Matten hergestellt.

Aufgrund der Neuheit und Fragilität dieser Vliese stellen die Sterilisierung und die nachträgliche Oberflächenmodifizierung der Vliesmaterialien und die Entwicklung von Verfahren zur Weiterverarbeitung und der Konfektionierung der Vliese ein ungelöstes technisches Problem dar, welches den kommerziellen Einsatz solcher Produkte bisher behindert. Bisher fehlen Verfahren zur Produktion von Halbzeugen definierter Größe und Geometrie. Insbesondere werden Verfahren gesucht, um aus flächigen Vliesen kleinere Abschnitte mit einigen Millimetern oder einigen 100 Mikrometern Kantenlange zu fertigen und daraus dreidimensionale Gebilde wie Hohlkugeln, Röhrchen und/oder Stäbchen zu formen. Solche Produkte sind für die Zellbesiedlung durch körpereigene Zellen des Empfängergewebes (z.B. Chondrozyten) in vitro und eine spätere Applikation vorteilhaft.

Bisher fehlen weiterhin Verfahren zur Applikation solcher Produkte, wobei ein klinischer und wirtschaftlicher Erfolg auf der Verwendbarkeit des neuartigen Materials sowie des neuartigen Materials nach Besiedlung mit lebenden Zellen zur Applikation mittels etablierter chirurgischer Methoden basieren muss.

Während die Abdeckung oberflächlicher Defekte z.B. der Haut oder des Knochens mit flächigen Vliesprodukten relativ gut gelingt, ist die Verfüllung von Kavitäten bzw. die Erreichbarkeit schwer zugänglicher Defekte im Knochen oder im Knorpel bisher ausgeschlossen. Es fehlen Produkte, die durch Injektion in Defekte eingebracht werden können oder durch Einpressen einer pastösen Substanz in den Defekt verfüllt werden und ortsständig verbleiben. Die operativen Verfahren sollen dabei z.B. den Routinen beim Einbringen konventioneller Knochenzemente verschiedener Viskosität (pastös bis injizierbar) entsprechen.

Problemlos gelingt die Erzeugung flächiger Vliese verschiedener Abmessungen, d.h. in einer dem abzudeckenden Defekt angepassten Größe. Möglich ist die Gestaltung gerichteter Strukturen durch die Verwendung strukturierter Vorlagen ("templates") oder eine geeignete Steuerung des elektrischen Feldes während des Spinnprozesses. Solche Gebilde können prinzipiell auch zu einfachen dreidimensionalen Konstrukten (z.B. Röhrchen) weiterverarbeitet werden (WO 2012/112564 A1, Agarwal, J.H. et al., Polymer, 2008, Band 49, S. 5603-5621; Buttafoco, L. et al., J. Control. Release, 2005, Band 101, S. 322-324; Yang, F. et al., Biomaterials, 2005, Band 26, S. 2603-2610; Lee, Y.-S. & Arinzeh, T.L., Polymers, 2011, Band 3, S. 413-426; Cui, W. et al., Sci. Technol. Adv. Mater., 2010, Band 11).

Verfahren zur Herstellung zusammenhängender Gebilde aus Vliesfragmenten sind nur aus anderen Gebieten der Technik bekannt. In der EP 0 603 774 A1 wird beispielsweise beschrieben, wie eine Mattenware für den Bereich der Heimtextilien aus Vliesfragmenten hergestellt werden kann. Dabei wird der Zusammenhalt durch Anschmelzen der Faseranteile erzielt. Die EP 0 371 847 A1 offenbart ein Verfahren, mit dem einzelne Filzstücke zu einem Verbundwerkstoff für Automobilformteile rekonstituiert werden können.

Eine Nutzung von Selbstorganisationseffekten zur Formbildung und die Gestaltung mikroskopischer Objekte ist bislang aus Nanofaservliesen oder daraus hergestellten Abschnitten oder Fragmenten nicht gelungen. Berichtet werden Verfahren zur Besiedlung von Vlieslagen im Bioreaktor mit anschließender Stapelung der besiedelten Schichten. Dadurch wird in gewisser Weise eine dreidimensionale, durch Zellen besiedelte, Struktur erreicht. Problematisch bleibt allerdings die Applikation solcher Produkte in Kavitäten und schwer zugangliche Defekte.

Eine erfolgreiche Zellbesiedlung vorgegebener, z.B. durch Schichtung von mehreren Vlieslagen erzeugten, dreidimensionalen Strukturen gelingt nur oberflächlich, da ein Einwachsen von Zellen in sehr kleinporige Strukturen (Größe < 200 bis 500 µm) ausgeschlossen ist und eine Versorgung von Zellen im Inneren der Konstrukte nicht gewährleistet werden kann.

Die Aufgabe war somit die Bereitstellung eines Verfahrens zur Herstellung einer dreidimensionalen, porösen Struktur aus Fragmenten, die aus mindestens einem Vlies aus Nanofasern besteht und für die Füllung von kleinporigen Strukturen, d.h. Strukturen einer Größe von kleiner als 200 bis 500 µm, geeignet ist.

Die Aufgabe wird gelöst durch das Verfahren gemäß Anspruch 1, die dreidimensionale, poröse Struktur gemäß Anspruch 11 und die Verwendung der dreidimensionalen, porösen Struktur gemäß Anspruch 17. Die abhängigen Ansprüche zeigen vorteilhafte Weiterbildungen auf.

Erfindungsgemäß wird somit ein Verfahren zur Herstellung einer dreidimensionalen, porösen Struktur aus Fragmenten, welche aus einem Vlies aus Nanofasern bestehen, umfassend
a) Schneiden von einem trockenen oder feuchten Vlies aus Nanofasern mit einem Laser in Fragmente und Suspendieren der Vliesfragmente in einem flüssigen Medium; oder
b) Schneiden von einem Vlies aus Nanofasern, das in einem flüssigen Medium vorliegt, mit einem Laser in Fragmente, wodurch eine Suspension von Vliesfragmenten in dem flüssigen Medium entsteht; und
c) Zumindest teilweises Entfernen von dem flüssigen Medium, wobei sich durch Selbstorganisation eine dreidimensionale, poröse Struktur aus Vliesfragmenten ausbildet.

Erfindungsgemäß wird unter dem Begriff "Fragment" ein kleines Stück bzw. ein kleiner Abschnitt von einem großen Vlies verstanden, wobei unter dem Begriff "Vlies" eine in einem kontinuierlichen Prozess erzeugte Bahn einer bestimmten Breite zu verstehen ist. Ein Vlies kann auch durch einen diskontinuierlichen Prozess erzeugt werden, der mit der vollständigen Belegung einer festgelegten Grundfläche abgeschlossen wird. Im Sinne der Erfindung werden hierbei Fragmente aus einem Vlies mittels Laser aus dem Vlies "herausgeschnitten" d.h. aus dem Vlies herausgetrennt. Diese Fragmente oder Stücke oder Abschnitte können nur auf diese Weise erzeugt werden, besitzen gegenüber den kompakten Vlies-Bahnen neue, zusätzliche Eigenschaften und bilden das Grundelement bzw. den Grundbaustein der hier beschriebenen Anwendung.

Erfindungsgemäß werden unter dem Begriff "Nanofasern" Fasern verstanden, die einen Durchmesser von 1 nm bis 10 µm aufweisen.

Durch den Entzug des flüssigen Mediums in Schritt c) wird die Adhäsion der Oberflächen benachbarter Vliesfragmente bewirkt und damit eine Ausbildung und Verfestigung einer dreidimensionalen, porösen Struktur erreicht. Es entsteht folglich eine mechanisch stabile Struktur.

Das trockene oder feuchte Vlies kann durch Elektrospinnen von Mikro- und Nanofasern hergestellt worden sein. Es kann sich dabei um ein Mikrofaservlies oder Nanofaservlies handeln.

Das Schneiden des Vlieses kann in einem kontinuierlichen oder diskontinuierlichen ("batch") Prozess stattfinden. Es können auch mehrere Vlieslagen aufeinander gelegt und gleichzeitig geschnitten werden.

Bei dem Laser kann es sich um einen Laser im kontinuierlichen Betrieb oder um einen Laser mit Pulsdauern im Nanosekundenbereich oder Ultrakurzpulsbereich (Pikosekunden bis Femtosekunden) handeln. Typische Wellenlängen des eingestrahlten Lichts sind in dem Bereich von 193 nm bis 1100 nm angesiedelt. Der Laser kann jedoch auch ein kontinuierlich oder gepulst arbeitender Laser im Infrarot-Bereich (d.h. λ im IR-Spektrum) sein (z.B. ein CO₂-Laser). Bevorzugt sendet der Laser Strahlung einer Wellenlänge von ca. 10 µm und einer Intensität ≥ 10 kW/cm² aus. Weiterhin bevorzugt ist ein Holmium-Laser oder ein Erbium-Laser.

Die Energiedichten bzw. Fluenzen, die beim Schneiden der trockenen oder feuchten Vliese mit gepulsten Lasern zur Anwendung kommen, liegen vorzugsweise im Bereich von 0,1 J/cm² bis 50 J/cm².

Die mittleren Leistungen der in Frage kommenden gepulsten Laser ist bevorzugt ≥ 100 mW.

Typische Schnittgeschwindigkeiten liegen zwischen 1 mm/s und 1000 mm/s, optional 10 mm/s und 100 mm/s.

Die Bildung der dreidimensionalen, porösen Struktur aus Vliesfragmenten über Selbstorganisation kann durch nicht-kovalente und/oder kovalente Wechselwirkungen bewirkt werden. Durch eine vor den Schritten a) oder b) durchgeführte, gezielte Oberflächenmodifizierung des Vlieses (z.B. Aktivierung, Erzeugung reaktionsfähiger Funktionalitäten) oder die Verwendung eines chemisch funktionalisierten Vlieses in Schritt a) oder b) kann beispielsweise eine Festigung der dreidimensionalen, porösen Struktur über (spezifische) kovalente chemische Bindungen erfolgen.

Entscheidender Vorteil des erfindungsgemäßen Verfahrens ist, dass durch die Verwendung eines Lasers Vliesfragmente geschaffen werden können, die über ein mechanisches Schneideverfahren nicht zugänglich wären. Bekannte mechanische Verfahren würden die vorteilhaften Nanostrukturen erst gar nicht entstehen lassen bzw. weitgehend schädigen. Die durch das erfindungsgemäße Verfahren mögliche Herstellung derartiger, hinreichend kleiner Vliesfragmente ohne Schädigung ihrer Mikro- und Nanofaserstruktur ist die Voraussetzung für die Erzielung der vorteilhaften Eigenschaften der erfindungsgemäßen dreidimensionalen, porösen Struktur.

Im Falle der Verwendung eines Lasers als Schneidewerkzeug bietet das erfindungsgemäße Verfahren - neben der Schneidefunktion - zudem die Möglichkeit der gezielten Oberflächenmodifizierung und Sterilisation des Produktes.

In dem erfindungsgemäßen Verfahren kann in Schritt c) ein Gel, eine Paste oder eine feste Struktur gebildet werden, insbesondere eine als biologische extrazelluläre Matrix dienende Struktur. Dies kann durch kontrollierten Entzug des flüssigen Mediums in dem erfindungsgemäßen Verfahren gesteuert werden, wobei durch den Entzug des Suspensionsmediums eine Anlagerung der Vliesfragmente und/oder Vliesfragmente und anhaftende Zellen bewirkt wird. Durch die Zugabe von biologischen Zellen in Schritt a) oder Schritt b) entsteht somit ein dreidimensionales Hybrid aus Vliesfragmenten und Zellen.

Die dreidimensionale Struktur des Gerüstes, insbesondere deren Porosität, wird durch Größe und Form der Vliesfragmente vorgegeben.

Durch fortschreitenden Entzug des Suspensionsmediums kann eine weitere Kompaktierung der dreidimensionalen Anordnung und eine Adhäsion der Substratoberflachen erfolgen, wodurch eine Verfestigung des Hybridsystems erreicht wird.

Das Vlies kann in Schritt a) oder b) des erfindungsgemäßen Verfahrens in vieleckige Vliesfragmente, Vliesfragmente mit abgerundeten Kanten, runde Vliesfragmente, dreieckige Vliesfragmente, quadratische Vliesfragmente, rechteckige Vliesfragmente, rhombische Vliesfragmente und/oder trapezförmige Vliesfragmente geschnitten werden.

Ferner können in Schritt a) oder Schritt b) Fragmente mit einer Kantenlänge von 50 µm bis 100 mm, optional 100 µm bis 10 mm, erzeugt werden. Bevorzugt werden Fragmente mit einer Fläche von ≤ 1 mm² erzeugt.

Nach Schritt a) oder Schritt b) können die Vliesfragmente und/oder nach Schritt c) kann die poröse, dreidimensionale Struktur mit biologischen Zellen, bevorzugt mit menschlichen Zellen, insbesondere mit Chondrozyten, Osteoblasten, Fibroblasten und/oder Stammzellen kontaktiert werden, wobei humane, embryonale Stammzellen ausgenommen sind. Hierbei dienen die einzelnen, im flüssigen Medium räumlich getrennt voneinander vorliegenden Fragmenten als Substrate für die Anlagerung und die Proliferation der Zellen.

Das erfindungsgemäße Verfahren kann dadurch gekennzeichnet sein, dass in Schritt a) die Vliesfragmente in Wasser, einer physiologischen Kochsalzlösung und/oder einem Nährmedium für Zellkulturen suspendiert werden oder in Schritt b) darin vorliegen. Der Vorteil eines feuchten Vlieses in Schritt a) oder Vlieses in einem flüssigen Medium in Schritt b) gegenüber einem trockenen Vlies in Schritt a) ist, dass sich erstere einfacher und effektiver suspendiert werden können.

Das Vlies kann in Schritt a) in einem gasförmigen Medium enthaltend oder bestehend aus Luft, Inertgas und/oder Prozessgas mit dem Laser bestrahlt werden. Ferner kann das Vlies in Schritt b) in einem flüssigen Medium enthaltend oder bestehend aus Wasser, physiologischer Kochsalzlösung und/oder Nährmedium für Zellkulturen vorliegen.

Bevorzugt wird in Schritt a) des Verfahrens ein Vlies verwendet, welches
i) Nanofasern mit einem Durchmesser von 10 nm bis 10 µm, bevorzugt mit einem Durchmesser von 50 nm bis 500 nm;
ii) Nanofasern aus biokompatiblen, resorbierbaren oder nichtresorbierbaren, synthetischen oder natürlichen Polymeren, bevorzugt Polymere ausgewählt aus der Gruppe bestehend aus Poly-L-Lactid, Poly-D-Lactid, Poly-(D,L)-Lactid, Poly-(L-Lactid-co-D,L-Lactid), Polyglycolsäure, Poly-(Lactid-co-Glycolid), Polyhydroxybutyrat und Poly-(Hydroxybutyrat-co-Hydroxyvalerat), sowie Mischungen hiervon;
iii) Nanofasern aus resorbierbaren, biokompatiblen, natürliche Polymeren, bevorzugt Kollagen, vernetztes Kollagen, Chitosan oder vergleichbare Materialien;
iv) bioaktive Füllstoffe, bevorzugt Hydroxyapatit und/oder Tricalciumphosphat, optional α-Tricalciumphosphat und/oder β-Tricalciumphosphat, sowie Mischungen hiervon;
v) Wirkstoffe, bevorzugt Antibiotika und/oder Wachstumsfaktoren; und/oder
vi) Additive, bevorzugt Farbstoffe, besonders bevorzugt Fluoreszenzfarbstoffe, insbesondere Chlorophyll;
enthält.

Vor dem Schritt a) oder Schritt b) des erfindungsgemäßen Verfahrens kann das Vlies aus Nanofasern mit einem Plasma, mit einem Laser, bevorzugt mit einem UV-Laser, oder mit UV-Strahlung behandelt werden. Hierbei kann insbesondere
i) das Vlies sterilisiert werden;
ii) die physikalischen und/oder chemischen Eigenschaften einer Oberfläche von dem Vlies zumindest bereichsweise modifiziert werden, wodurch insbesondere ein zumindest bereichsweise hydrophiles und/oder hydrophobes Vlies entsteht;
iii) die physikalischen und/oder chemischen Eigenschaften einer Vlies-Oberseite und Vlies-Unterseite zumindest bereichsweise dergestalt modifiziert werden, dass ein amphiphiles Vlies entsteht, und/oder
iv) das Vlies an seiner Oberfläche, insbesondere durch Plasmapolymerisation, chemisch funktionalisiert werden.

Ein Vorteil der chemischen Hydrophobisierung, Hydrophilisierung, Amphiphilisierung und/oder Modifizierung mit funktionellen Gruppen ist die gezielte Anpassung der über das Verfahren hergestellten dreidimensionalen, porösen Struktur aus Vliesfragmenten an ein bestimmtes Ziel ("target"), also z.B. bei der Verwendung der Struktur als Füllstoff an die Eigenschaften eines bestimmten, zu füllenden Materials. Ferner kann somit eine dreidimensionale, poröse Struktur generiert werden, die durch kovalente chemische Bindungen (innerhalb von einem Vliesfragment und/oder zwischen verschiedenen Vliesfragmenten) verstärkt wird bzw. verstärkbar ist. In dieser Hinsicht kann z.B. eine thermische Aushärtung der Struktur zu einer intermolekularen Vernetzung von Molekülen gleicher und/oder verschiedener Vliesfragmente bewirkt werden, nachdem die Struktur als Füllmaterial in einen Knochen gegeben wurde.

Durch gezielte Einstellung bzw. Wahl der Fragmentierung in Schritt a) oder Schritt b), durch Wahl des flüssigen Mediums in Schritt a) oder Schritt b) und/oder durch Wahl einer Vorbehandlung des Vlieses mit Plasma vor Schritt a) oder Schritt b), können die Vliespartikel nach der Suspendierung gezielt folgende Struktur annehmen:
i) eine gestreckte, plättchenförmige Struktur;
ii) eine kugelförmige Struktur, bevorzugt Micellen;
iii) eine zylindrische Struktur, bevorzugt Mikrotubes; und/oder
iv) Mischungen oder Aggregate dieser Strukturen.

Erfindungsgemäß wird ferner eine dreidimensionale, poröse Struktur bereitgestellt, die Fragmente aus mindestens einem Vlies aus Nanofasern enthält oder daraus besteht, wobei die Fragmente eine Kantenlänge von 50 µm bis 100 mm und/oder eine Fläche von ≤ 1 mm² aufweisen. Optional kann die Kantenlänge in dem Bereich von 100 µm bis 10 mm angesiedelt sein.

Die dreidimensionale, poröse Struktur kann dadurch gekennzeichnet sein, dass die Fragmente aus dem mindestens einen Vlies aus Nanofasern zumindest teilweise und/oder bereichsweise mindestens eine Schnittkante aufweisen, welche durch Laserstrahlung geschnitten worden sind.

Die dreidimensionale, poröse Struktur kann biologische Zellen aufnehmen bzw. aufweisen, bevorzugt menschliche Zellen, insbesondere Chondrozyten, Osteoblasten, Fibroblasten und/oder Stammzellen, wobei humane embryonale Stammzellen ausgenommen sind. Bevorzugt sind diese Zellen an zumindest einer Oberfläche der Struktur gebunden. Die Bindung kann über nicht-kovalente und/oder kovalente chemische Wechselwirkungen erfolgen, wobei nicht-kovalente Wechselwirkungen bevorzugt sind.

Die erfindungsgemäße dreidimensionale, poröse Struktur kann nach dem erfindungsgemäßen Verfahren herstellbar sein bzw. hergestellt werden. Optional kann die dreidimensionale, poröse Struktur in einem flüssigen Medium suspendiert sein.

Bevorzugt wird die dreidimensionale, poröse Struktur in der Medizin, insbesondere der regenerativen Medizin, verwendet. Bevorzugt wird die dreidimensionale, poröse Struktur per Injektion appliziert.

Besonders bevorzugt wird die dreidimensionale, poröse Struktur in der Behandlung von Gewebeschäden, bevorzugt Knochenschäden, Knorpelschäden, Bandscheiben und/oder Hautschäden, verwendet.

Darüberhinaus kann die dreidimensionale, poröse Struktur als Substrat für die Immobilisierung von biologischen Zellen verwendet werden. Bevorzugt sind hierbei menschlichen Zellen, insbesondere Chondrozyten, Osteoblasten, Fibroblasten und/oder Stammzellen, wobei humane embryonale Stammzellen ausgenommen sind.

Letztlich eignet sich die dreidimensionale, poröse Struktur für die Konstruktion von biologischem Gewebe und/oder als biologischen Füllstoff, bevorzugt als Füllstoff für Knochen, Knorpel und/oder Haut.

Ferner wird vorgeschlagen, die erfindungsgemäße, poröse Struktur in vitro in zu einem der oben genannten Zwecke zu verwenden.

Anhand der nachfolgenden Beispiele soll der erfindungsgemäße Gegenstand näher erläutert werden, ohne diesen auf die hier dargestellten spezifischen Ausführungsformen einschränken zu wollen.

### Beispiel 1 - Herstellung verschiedener Formen der dreidimensionalen, porösen Struktur aus Vliesfragmenten

Die Anwendbarkeit der erfindungsgemäßen dreidimensionalen Struktur für die Zellbesiedlung und die Verwendung als per Injektion implantierbares Produkt sind Gestalt, spezifische Oberflächen und Größe der Vliespartikel. Diese Schlüsselparameter sind abhängig einerseits von den chemischen und physikalischen Eigenschaften des verwendeten Vlieses und andererseits von den gewählten Prozessparametern bei der Plasmabehandlung und dem Laserschneiden des Vlieses bzw. der Vliesfragmente.

Beispielhaft erfolgt vor der Konfektionierung der Mikro- und Nanofaservliese durch Laser eine Plasmabehandlung zur Modifizierung der physikalischen und chemischen Eigenschaften der Faseroberflachen bzw. der Vliesoberflächen bzw. eine Oberflächenfunktionalisierung durch Plasmapolymerisation.

Durch Wahl von Form, Größe und Seitenlängenverhältnis der Vliesfragmente und/oder der Oberflächeneigenschaften bzw. Oberflächenfunktionalisierung (z.B. hydrophil, hydrophob, amphiphil) sowie des Suspensionsmediums und der Konzentration können die Fragmente in diesen Suspensionen verschiedene, gezielt einstellbare, dreidimensionale Strukturen bilden. Insbesondere sind flächige (Plättchen), zylindrische (Stäbchen und Röhrchen) und kugelförmige (Kugeln und Hohlkugeln) Gebilde herstellbar.

Beispielsweise sind folgende Gestaltvariationen der Vliespartikel (Fragmente) einstellbar: gestreckte, plättchenförmige Gestalt der Vlies-Fragmente bei guter Verträglichkeit von Vliesoberfläche und Suspensionsmedium, kugelförmige Gebilde (Mizellen) durch Minimierung der Oberfläche bei Unverträglichkeit von Polymeroberfläche und Suspensionsmedium, Mikrotubes durch Aufrollen der Vliese bei amphiphiler Oberflächenausgestaltung und Verwendung rechteckiger Fragmente bei hohem Seitenlängenverhältnis, sowie eine Aggregation der oben genannten Strukturen zu größeren Einheiten.

### Beispiel 2 -Struktur aus Kollagenfaser-Vliesfragmenten und Osteozyten bzw. Chondrozyten und deren Verwendung

Injizierbare dreidimensionale, poröse Strukturen aus Nanokollagenfasern und Osteozyten sind besonders geeignet, um schwer erreichbare Knochendefektzonen auszufüllen. Dies kann beim nicht hundertprozentig passgerechten Rekonstruktionseingriff nötig sein, wenn kleine Restfugen mit diesem Material ausgefüllt werden. Dies ist besonders bei kritischen Operationen wie z. B. Operationen im septischen Bereich indiziert.

Aber auch schwer erreichbare knöcherne Defekte bei Rekonstruktionsoperationen stellen eine medizinische Indikation dar, besonders dann, wenn die Knochenproduktion angeregt werden muss.

Im Bereich des Knorpels sind alle osteoarthritischen Knorpeldefekte zu erwähnen, die mittels orthoskopischer Verfahren angegangen werden können. Hier kann eine dreidimensionale, poröse Struktur aus Kollagenfaser-Vliesfragmenten und Chondrozyten in arthroskopisch vorbereitete Knorpeldefekte eingegeben werden. Dabei ist besonders an Defekte in schwer zugänglichen Gelenken zu denken, wie z. B. Huft-, Schulter- oder Sprunggelenk.

Selbstverständlich ist auch das Kniegelenk für einen solchen injizierten Zell-Kollagen-Verbund ein gutes Zielorgan. Hier bietet das erfindungsgemäße Material und die durch die Eigenschaften des erfindungsgemäßen Materials eröffnete Applikationsform der Injektion eine Verbesserung zu den bisherigen Verfahren, bei denen sogenannte 3D-Konstrukte als Zell-Polymer-Hybride eingebracht werden.

## Patentansprüche

1. Verfahren zur Herstellung einer dreidimensionalen, porösen Struktur aus Fragmenten, welche aus einem Vlies aus Nanofasern bestehen, umfassend
a) Schneiden von einem trockenen oder feuchten Vlies aus Nanofasern mit einem Laser in Fragmente und Suspendieren der Vliesfragmente in einem flüssigen Medium; oder
b) Schneiden von einem Vlies aus Nanofasern, das in einem flüssigen Medium vorliegt, mit einem Laser in Fragmente, wodurch eine Suspension von Vliesfragmenten in dem flüssigen Medium entsteht; und
c) Zumindest teilweises Entfernen von dem flüssigen Medium, wobei sich durch Selbstorganisation eine dreidimensionale, poröse Struktur aus Vliesfragmenten ausbildet.

2. Verfahren gemäß dem vorhergehenden Anspruch, **dadurch gekennzeichnet, dass** in Schritt c) ein Gel, eine Paste oder eine feste Struktur, insbesondere eine als biologische extrazelluläre Matrix dienende Struktur, gebildet wird.

3. Verfahren gemäß einem der vorhergehenden Ansprüche, **dadurch gekennzeichnet, dass** in Schritt a) oder Schritt b) das Vlies in vieleckige Vliesfragmente, Vliesfragmente mit abgerundeten Kanten, runde Vliesfragmente, dreieckige Vliesfragmente, quadratische Vliesfragmente, rechteckige Vliesfragmente, rhombische Vliesfragmente und/oder trapezförmige Vliesfragmente geschnitten wird.

4. Verfahren gemäß einem der vorhergehenden Ansprüche, **dadurch gekennzeichnet, dass** in Schritt a) oder Schritt b) Fragmente mit einer Kantenlänge von 50 µm bis 100 mm, optional 100 µm bis 10 mm und/oder mit einer Fläche von ≤ 1 mm² erzeugt werden.

5. Verfahren gemäß einem der vorhergehenden Ansprüche, **dadurch gekennzeichnet, dass** nach Schritt a) oder Schritt b) die Vliesfragmente und/oder nach Schritt c) die poröse, dreidimensionale Struktur mit biologischen Zellen, bevorzugt mit menschlichen Zellen, insbesondere mit Chondrozyten, Osteoblasten, Fibroblasten und/oder Stammzellen ausgenommen humanen, embryonalen Stammzellen, kontaktiert wird/werden.

6. Verfahren gemäß einem der vorhergehenden Ansprüche, **dadurch gekennzeichnet, dass** das Vlies in Schritt a) in einem gasförmigen Medium enthaltend oder bestehend aus Luft, Inertgas und/oder Prozessgas mit dem Laser bestrahlt wird und/oder das Vlies in Schritt b) in einem flüssigen Medium enthaltend oder bestehend aus Wasser, physiologischer Kochsalzlösung und/oder Nährmedium für Zellkulturen vorliegt.

7. Verfahren gemäß einem der vorhergehenden Ansprüche, **dadurch gekennzeichnet, dass** in Schritt a) das Vlies in einem gasförmigen oder flüssigen Medium, bevorzugt Luft, Inertgas, Prozessgas, Wasser, physiologische Kochsalzlösung und Nährmedium für Zellkulturen, mit dem Laser bestrahlt wird.

8. Verfahren gemäß einem der vorhergehenden Ansprüche, **dadurch gekennzeichnet, dass** in Schritt a) ein Vlies verwendet wird, welches
i) Nanofasern mit einem Durchmesser von 10 nm bis 10 µm, bevorzugt mit einem Durchmesser von 50 nm bis 500 nm;
ii) Nanofasern aus biokompatiblen, resorbierbaren oder nichtresorbierbaren, synthetischen oder natürlichen Polymeren, bevorzugt Polymere ausgewählt aus der Gruppe bestehend aus Poly-L-Lactid, Poly-D-Lactid, Poly-(D,L)-Lactid, Poly-(L-Lactid-co-D,L-Lactid), Polyglycolsäure, Poly-(Lactid-co-Glycolid), Polyhydroxybutyrat und Poly-(Hydroxybutyrat-co-Hydroxyvalerat), sowie Mischungen hiervon;
iii) Nanofasern aus resorbierbaren, biokompatiblen, natürliche Polymeren, bevorzugt Kollagen, vernetztes Kollagen, Chitosan oder vergleichbare Materialien;
iv) bioaktive Füllstoffe, bevorzugt Hydroxyapatit und/oder Tricalciumphosphat, optional α-Tricalciumphosphat und/oder β-Tricalciumphosphat, sowie Mischungen hiervon;
v) Wirkstoffe, bevorzugt Antibiotika und/oder Wachstumsfaktoren; und/oder
vi) Additive, bevorzugt Farbstoffe, besonders bevorzugt Fluoreszenzfarbstoffe, insbesondere Chlorophyll;
enthält.

9. Verfahren gemäß einem der vorhergehenden Ansprüche, **dadurch gekennzeichnet, dass** vor Schritt a) oder Schritt b) das Vlies aus Nanofasern mit einem Plasma, mit einem Laser, bevorzugt einem UV-Laser, oder mit UV-Strahlung behandelt wird, wobei insbesondere
i) das Vlies sterilisiert wird;
ii) die physikalischen und/oder chemischen Eigenschaften einer Oberfläche von dem Vlies zumindest bereichsweise modifiziert wird, wodurch insbesondere ein zumindest bereichsweise hydrophiles und/oder hydrophobes Vlies entsteht;
iii) die physikalischen und/oder chemischen Eigenschaften einer Vlies-Oberseite und Vlies-Unterseite zumindest bereichsweise dergestalt modifiziert werden, dass ein amphiphiles Vlies entsteht, und/oder
iv) das Vlies an seiner Oberfläche, insbesondere durch Plasmapolymerisation, chemisch funktionalisiert wird.

10. Verfahren gemäß einem der vorhergehenden Ansprüche, **dadurch gekennzeichnet, dass** durch Wahl der Fragmentierung in Schritt a) oder Schritt b), durch Wahl des flüssigen Mediums in Schritt a) oder Schritt b) und/oder durch Wahl einer Vorbehandlung des Vlieses mit Plasma vor Schritt a) oder Schritt b) die Vliespartikel nach der Suspendierung gezielt folgende Struktur annehmen:
i) eine gestreckte, plättchenförmige Struktur;
ii) eine kugelförmige Struktur, bevorzugt Micellen;
iii) eine zylindrische Struktur, bevorzugt Mikrotubes; und/oder
iv) Mischungen oder Aggregate dieser Strukturen.

11. Dreidimensionale, poröse Struktur, die Fragmente aus mindestens einem Vlies aus Nanofasern enthält oder daraus besteht, wobei die Fragmente eine Kantenlänge von 50 µm bis 100 mm und/oder eine Fläche von ≤ 1 mm² aufweisen.

12. Dreidimensionale, poröse Struktur gemäß Anspruch 11, **dadurch gekennzeichnet, dass** die Fragmente aus dem mindestens einen Vlies aus Nanofasern zumindest teilweise und/oder bereichsweise mindestens eine Schnittkante aufweisen, welche durch Laserstrahlung geschnitten worden sind.

13. Dreidimensionale, poröse Struktur gemäß einem der Ansprüche 11 bis 12, **dadurch gekennzeichnet, dass** die dreidimensionale, poröse Struktur biologische Zellen, bevorzugt menschliche Zellen, insbesondere Chondrozyten, Osteoblasten, Fibroblasten und/oder Stammzellen, ausgenommen humane, embryonale Stammzellen, aufweist, wobei diese Zellen bevorzugt an zumindest einer Oberfläche der Struktur gebunden sind.

14. Dreidimensionale, poröse Struktur gemäß einem der Ansprüche 11 bis 13 und herstellbar nach dem Verfahren gemäß einem der Ansprüche 1 bis 10.

15. Dreidimensionale, poröse Struktur gemäß einem der Ansprüche 11 bis 14 zur Verwendung in der Medizin, insbesondere der regenerativen Medizin.

16. Dreidimensionale, poröse Struktur gemäß einem der Ansprüche 11 bis 14 zur Verwendung
a) in der Behandlung von Gewebeschäden, bevorzugt Knochenschäden, Knorpelschäden, Bandscheiben und/oder Hautschäden;
b) in der Immobilisierung von biologischen Zellen, bevorzugt menschlichen Zellen, insbesondere Chondrozyten, Osteoblasten, Fibroblasten und/oder Stammzellen, wobei humane embryonale Stammzellen ausgenommen sind; und/oder
c) in der Konstruktion von biologischem Gewebe; und/oder
d) als biologischer Füllstoff, bevorzugt als Füllstoff für Knochen, Knorpel und/oder Haut.

17. *In-vitro*-Verwendung der dreidimensionalen, porösen Struktur gemäß einem der Ansprüche 11 bis 14
a) in der Behandlung von Gewebeschäden, bevorzugt Knochenschäden, Knorpelschäden, Bandscheiben und/oder Hautschäden;
b) zur Immobilisierung von biologischen Zellen, bevorzugt menschlichen Zellen, insbesondere Chondrozyten, Osteoblasten, Fibroblasten und/oder Stammzellen, wobei humane embryonale Stammzellen ausgenommen sind; und/oder
c) in der Konstruktion von biologischem Gewebe; und/oder
d) als biologischer Füllstoff, bevorzugt als Füllstoff für Knochen, Knorpel und/oder Haut.

## Claims

1. Method for the production of a three-dimensional porous structure made of fragments which consist of a web made of nanofibres, comprising
a) cutting a dry or wet web made of nanofibres into fragments with a laser and suspending the web fragments in a liquid medium; or
b) cutting a web made of nanofibres which is present in a liquid medium into fragments with a laser, as a result of which a suspension of web fragments in the liquid medium is produced; and
c) at least partial removal of the liquid medium, a three-dimensional, porous structure being formed from web fragments by means of self-organisation.

2. Method according to the preceding claim, **characterised in that**, in step c), a gel, a paste or a solid structure, in particular a structure serving as biological extracellular matrix is formed.

3. Method according to one of the preceding claims, **characterised in that**, in step a) or step b), the web is cut into polygonal web fragments, web fragments with rounded edges, round web fragments, triangular web fragments, square web fragments, rectangular web fragments, rhomboid web fragments and/or trapezoidal web fragments.

4. Method according to one of the preceding claims, **characterised in that**, in step a) or step b), fragments with an edge length of 50 µm to 100 mm, optionally 100 µm to 10 mm, and/or with a surface area ≤ 1 mm² are produced.

5. Method according to one of the preceding claims, **characterised in that**, after step a) or step b), the web fragments and/or, after step c), the porous, three-dimensional structure is/are contacted with biological cells, preferably with human cells, in particular with chondrocytes, osteoblasts, fibroblasts and/or stem cells, with the exception of human embryonic stem cells.

6. Method according to one of the preceding claims, **characterised in that** the web, in step a), contained in a gaseous medium or consisting of air, inert gas and/or process gas, is irradiated with the laser, and/or the web, in step b), is present, contained in a liquid medium or consisting of water, physiological common salt solution and/or nutrient medium for cell culture.

7. Method according to one of the preceding claims, **characterised in that**, in step a), the web is irradiated with the laser in a gaseous or liquid medium, preferably air, inert gas, process gas, water, physiological common salt solution and nutrient medium for cell cultures.

8. Method according to one of the preceding claims, **characterised in that**, in step a), a web is used which comprises
i) nanofibres with a diameter of 10 nm to 10 µm, preferably with a diameter of 50 nm to 500 nm;
ii) nanofibres made of biocompatible, resorbable or non-resorbable, synthetic or natural polymers, preferably polymers selected from the group consisting of poly-L-lactide, poly-D-lactide, poly-(D,L)-lactide, poly-(L-lactide-co-D,L-lactide), polyglycolic acid, poly-(lactide-co-glycolide), polyhydroxybutyrate and poly-(hydroxybutyrate-co-hydroxyvalerate), and also mixtures hereof;
iii) nanofibres made of resorbable, biocompatible, natural polymers, preferably collagen, crosslinked chitosan or comparable materials, or
iv) bioactive fillers, preferably hydroxyapatite and/or tricalcium phosphate, optionally α-tricalcium phosphate and/or β-tricalcium phosphate, and also mixtures hereof;
v) active substances, preferably antibiotics and/or growth factors; and/or
vi) additives, preferably colourants, particularly preferably fluorescent dyes, in particular chlorophyll.

9. Method according to one of the preceding claims, **characterised in that**, before step a) or step b), the web made of nanofibres is treated with a plasma, with a laser, preferably a UV laser, or with UV radiation, in particular
i) the web being sterilised;
ii) the physical and/or chemical properties of a surface of the web being modified at least in regions, as a result of which in particular a hydrophilic and/or hydrophobic web is produced at least in regions;
iii) the physical and/or chemical properties of a web upper side and web underside being modified at least in regions such that an amphiphilic web is produced, and/or
iv) the web being chemically functionalised on its surface, in particular by plasma polymerisation.

10. Method according to one of the preceding claims, **characterised in that**, by the choice of the fragmentation in step a) or step b), by the choice of the liquid medium in step a) or step b) and/or by the choice of a pretreatment of the web with plasma before step a) or b), the web particles assume specifically the following structure after being suspended:
i) an elongated, plate-shaped structure;
ii) a spherical structure, preferably micelles;
iii) a cylindrical structure, preferably microtubes; and/or
iv) mixtures or aggregates of these structures.

11. Three-dimensional, porous structure which comprises fragments of at least one web made of nanofibres or consists thereof, the fragments having an edge length of 50 µm to 100 mm and/or a surface area of ≤ 1 mm².

12. Three-dimensional, porous structure according to claim 11, **characterised in that** the fragments of the at least one web made of nanofibres have at least one cut edge, at least partially and/or in regions, which was cut by laser radiation.

13. Three-dimensional, porous structure according to one of the claims 11 to 12, **characterised in that** the three-dimensional, porous structure has biological cells, preferably human cells, in particular chondrocytes, osteoblasts, fibroblasts and/or stem cells, with the exception of human embryonic stem cells, these cells being bonded preferably to at least one surface of the structure.

14. Three-dimensional, porous structure according to one of the claims 11 to 13 and producible according to the method according to one of the claims 1 to 10.

15. Three-dimensional, porous structure according to one of the claims 11 to 14 for use in medicine, in particular in regenerative medicine.

16. Three-dimensional, porous structure according to one of the claims 11 to 14 for use
a) in the treatment of tissue damage, preferably bone damage, cartilage damage, intervertebral discs and/or skin damage;
b) in the immobilisation of biological cells, preferably human cells, in particular chondrocytes, osteoblasts, fibroblasts and/or stem cells, human embryonic stem cells being the exception; and/or
c) in the construction of biological tissue; and/or
d) as biological filler, preferably as filler for bones, cartilage and/or skin.

17. *In-vitro* use of the three-dimensional, porous structure according to one of the claims 11 to 14
a) in the treatment of tissue damage, preferably bone damage, cartilage damage, intervertebral discs and/or skin damage;
b) for the immobilisation of biological cells, preferably human cells, in particular chondrocytes, osteoblasts, fibroblasts and/or stem cells, human embryonic stem cells being the exception; and/or
c) in the construction of biological tissue; and/or
d) as biological filler, preferably as filler for bones, cartilage and/or skin.

## Revendications

1. Procédé de fabrication d'une structure poreuse tridimensionnelle à partir de fragments qui sont constitués d'un voile de nanofibres, comprenant
a) la découpe en fragments à l'aide d'un laser d'un voile sec ou humide de nanofibres, et la mise en suspension des fragments de voile dans un milieu liquide ; ou
b) la découpe en fragments à l'aide d'un laser d'un voile de nanofibres se présentant dans un milieu liquide, en créant de ce fait une suspension de fragments de voile dans le milieu liquide ; et
c) l'enlèvement au moins partiel à partir du milieu liquide, en réalisant de ce fait par auto-organisation une structure poreuse tridimensionnelle de fragments de voile.

2. Procédé selon la revendication précédente, **caractérisé en ce que**, dans l'étape c), il y a formation d'un gel, d'une pâte ou d'une structure solide, en particulier d'une structure servant de matrice extracellulaire biologique.

3. Procédé selon l'une des revendications précédentes, **caractérisé en ce que**, dans l'étape a) ou dans l'étape b), le voile est découpé en fragments de voile polygonaux, en fragments de voile à arêtes arrondies, en fragments de voile circulaires, en fragments de voile triangulaires, en fragments de voile carrés, en fragments de voile rectangulaires, en fragments de voile en forme de losange et/ou en fragments de voile trapézoïdaux.

4. Procédé selon l'une des revendications précédentes, **caractérisé en ce que**, dans l'étape a) ou dans l'étape b), il y a production de fragments ayant une longueur d'arête de 50 µm à 100 mm, en option de 100 µm à 10 mm, et/ou avec une aire ≤ 1 mm².

5. Procédé selon l'une des revendications précédentes, **caractérisé en ce que**, après l'étape a) ou après l'étape b), les fragments de voile et/ou, après l'étape c), la structure tridimensionnelle poreuse, sont mis en contact avec des cellules biologiques, de préférence avec des cellules humaines, en particulier avec des chondrocytes, des ostéoblastes, des fibroblastes et/ou des cellules souches, à l'exception des cellules souches embryonnaires humaines.

6. Procédé selon l'une des revendications précédentes, **caractérisé en ce que** le voile, dans l'étape a), est irradié par un laser dans un milieu gazeux contenant de l'air, un gaz inerte et/ou un gaz de procédé ou en étant constitué, et/ou le voile, dans l'étape b), se présente dans un milieu liquide contenant de l'eau, une solution salée physiologique et/ou un milieu nutritif pour cultures de cellules, ou en est constitué.

7. Procédé selon l'une des revendications précédentes, **caractérisé en ce que**, dans l'étape a), le voile est irradié par le laser dans un milieu gazeux ou liquide, de préférence l'air, un gaz inerte, un gaz de procédé, l'eau, une solution salée physiologique et un milieu nutritif pour cultures de cellules.

8. Procédé selon l'une des revendications précédentes, **caractérisé en ce que**, dans l'étape a), on utilise un voile qui contient
i) des nanofibres ayant un diamètre de 10 nm à 10 µm, de préférence un diamètre de 50 nm à 500 nm ;
ii) des nanofibres obtenues à partir de polymères biocompatibles, résorbables ou non-résorbables, synthétiques ou naturels, de préférence des polymères choisis dans le groupe consistant en le poly-L-lactide, le poly-D-lactide, le poly-(D,L)-lactide, le poly-(L-lactide-co-D,L-lactide), le poly(acide glycolique), le poly-(lactide-co-glycolide), le polyhydroxybutyrate et le poly-(hydroxybutyrate-co-hydroxyvalérate), ainsi que les mélanges de ceux-ci ;
iii) des nanofibres obtenues à partir de polymères résorbables, biocompatibles, naturelles, de préférence le collagène, le collagène à réticulation croisée, le chitosan ou les matériaux comparables ;
iv) des charges bioactives, de préférence l'hydroxyapatite et/ou le phosphate tricalcique, en option le phosphate tricalcique α et/ou le phosphate tricalcique β, ainsi que les mélanges de ceux-ci ;
v) des principes actifs, de préférence des antibiotiques et/ou des facteurs de croissance ; et/ou
vi) des additifs, en particulier des colorants, d'une manière particulièrement préférée des colorants fluorescents, en particulier la chlorophylle.

9. Procédé selon l'une des revendications précédentes, **caractérisé en ce que**, avant l'étape a) ou avant l'étape b), le voile de nanofibres est traité avec un plasma, avec un laser, de préférence un laser UV, ou avec un rayonnement UV, auquel cas en particulier
i) on stérilise le voile ;
ii) on modifie au moins par zones les propriétés physiques et/ou chimiques d'une surface du voile, ce en conséquence de quoi il se forme en particulier un voile au moins par zones hydrophile et/ou hydrophobe ;
iii) on modifie au moins par zones les propriétés physiques et/ou chimiques d'une face supérieure du voile et d'une face inférieure du voile, de façon qu'il se crée un voile amphiphile, et/ou
iv) on soumet à une fonctionnalisation chimique le voile sur sa surface, en particulier par polymérisation par plasma.

10. Procédé selon l'une des revendications précédentes, **caractérisé en ce que**, sous l'effet du choix de la fragmentation dans l'étape a) ou dans l'étape b), du choix du milieu liquide dans l'étape a) ou dans l'étape b), et/ou du choix d'un prétraitement du voile avec un plasma avant l'étape a) ou avant l'étape b), les particules de voile prennent d'une manière ciblée, après la mise en suspension, la structure suivante :
i) une structure lamellaire étirée ;
ii) une structure sphérique, de préférence des micelles ;
iii) une structure cylindrique, de préférence des microtubes ; et/ou
iv) des mélanges sous agrégat de ces structures.

11. Structure poreuse tridimensionnelle, qui contient des fragments d'au moins un voile de nanofibres ou en est constituée, les fragments présentant une longueur d'arête de 50 µm à 100 mm et/ou une aire ≤ 1 mm².

12. Structure poreuse tridimensionnelle selon la revendication 11, **caractérisée en ce que** les fragments d'au moins un voile de nanofibres présentent au moins partiellement et/ou par zone au moins une arête de coupe, qui a été découpée par un rayonnement laser.

13. Structure poreuse tridimensionnelle selon l'une des revendications 11 à 12, **caractérisée en ce que** la structure poreuse tridimensionnelle présente des cellules biologiques, de préférence des cellules humaines, en particulier des chondrocytes, des ostéoblastes, des fibroblastes et/ou des cellules souches, à l'exception des cellules souches embryonnaires humaines, ces cellules étant de préférence liées à au moins une surface de la structure.

14. Structure poreuse tridimensionnelle selon l'une des revendications 11 à 13, et pouvant être fabriquée par le procédé selon l'une des revendications 1 à 10.

15. Structure poreuse tridimensionnelle selon l'une des revendications 11 à 14 pour une utilisation en médecine, en particulier en médecine régénérative.

16. Structure poreuse tridimensionnelle selon l'une des revendications 11 à 14, pour une utilisation
a) dans le traitement de lésions tissulaires, de préférence des lésions osseuses, des lésions cartilagineuses, des disques intervertébraux et/ou des lésions cutanées ;
b) dans l'immobilisation de cellules biologiques, de préférence des cellules humaines, en particulier des chondrocytes, des ostéoblastes, des fibroblastes et/ou des cellules souches, auquel cas sont exclues les cellules souches embryonnaires humaines ; et/ou
c) dans la construction de tissus biologiques ; et/ou
d) en tant que matière de remplissage biologique, de préférence en tant que matière de remplissage pour des os, du cartilage et/ou la peau.

17. Utilisation *in vitro* de la structure poreuse tridimensionnelle selon l'une des revendications 11 à 14
a) dans le traitement de lésions tissulaires, de préférence des lésions osseuses, des lésions cartilagineuses, des disques intervertébraux et/ou des lésions cutanées ;
b) dans l'immobilisation de cellules biologiques, de préférence des cellules humaines, en particulier des chondrocytes, des ostéoblastes, des fibroblastes et/ou des cellules souches, auquel cas sont exclues les cellules souches embryonnaires humaines ; et/ou
c) dans la construction de tissus biologiques ; et/ou
d) en tant que matière de remplissage biologique, de préférence en tant que matière de remplissage pour des os, du cartilage et/ou la peau.
